# EUROPEAN PATENT APPLICATION

(11) **EP 4 502 184 A1**
(43) Date of publication of application: **05.02.2025**
(21) Application number: 23788316.0
(22) Date of filing: 10.04.2023
(51) Int. Cl.: C12Q 1/6886, A61K 31/7088, A61K 48/00, A61P 15/00, A61P 35/00, C07K 14/705, C07K 14/71, C12N 5/09, C12N 15/09, C12N 15/113, C12Q 1/06, C12Q 1/6851, C12Q 1/686, G01N 33/50, G01N 33/53, G01N 33/574

(54) **METHOD TO ASSIST IN SCREENING FOR LATE RECURRENCE OF ER+ BREAST CANCER, AND METHOD FOR SCREENING THERAPEUTIC DRUGS**

(30) Priority: 11.04.2022 JP 2022065345
(71) Applicant: Japanese Foundation For Cancer Research, Tokyo 135-8550 (JP)
(72) Inventor: FUKUOKA Megumi, Tokyo 135-8550 (JP); ICHIKAWA Yuichi, Tokyo 135-8550 (JP); OSAKO Tomo, Tokyo 135-8550 (JP); FUJITA Tomoko, Tokyo 135-8550 (JP); UENO Takayuki, Tokyo 135-8550 (JP); SAITOH Noriko, Tokyo 135-8550 (JP)
(74) Representative: Prüfer & Partner mbB Patentanwälte · Rechtsanwälte
(86) International application number: PCT/JP2023/014616
(87) International publication number: WO 2023/199898

(57) **Abstract**

There are many cases of late recurrent breast cancer that recurs after 5 years or more after surgery. If a marker for determining a late recurrence risk can be found, it can lead to early detection and treatment of recurrence. The present inventors analyzed clinical specimens and found that the ELEANOR expression correlates with the late recurrence of breast cancers. The risk of late recurrence can be tested by analyzing the ELEANOR expression in a breast cancer primary lesion. It is also possible to screen therapeutic drugs for breast cancers by using ELEANOR expression as an indicator. Furthermore, siRNA and LNA that suppress ELEANOR expression can also function as therapeutic drugs for breast cancers.

## Description

### Technical Field

The present invention relates to a marker for predicting recurrence of a breast cancer and a test kit and particularly relates to a marker for predicting late recurrence of an ER-positive breast cancer. In addition, the present invention relates to a method for screening therapeutic drugs using the marker as an indicator.

### Background Art

The number of breast cancer cases is on the rise both at home and abroad. In Japan, the number one cause of cancers in women is a breast cancer, approximately 94,000 people (2018 statistics) have developed, and approximately 15,000 people (2019 statistics) have died. There are many breast cancer patients worldwide, with the number of patients exceeding 2.2 million in 2020.

Breast cancers are treated with surgery, radiation therapy, and chemotherapy as in other cancers, and the basic treatment is to remove the cancer through surgery. Breast cancers are classified, in addition to staging, into subtypes based on the cancer cell characteristics such as the expression of hormone receptors: estrogen receptor (ER), progesterone receptor (PgR), and epidermal growth factor receptor type 2 (HER2), and treatment plans are established. If a hormone receptor is positive, endocrine therapy is often effective.

Approximately 70% of breast cancers are an ER-positive type expressing ER, and endocrine therapy that inhibits estrogen function is effective. However, if the treatment extends over a long period of time, resistance to the treatment occurs, which frequently leads to recurrence. It is known that although an ER-negative subtype often recurs within 3 years, in an ER-positive subtype, the risk of recurrence continues for 5 years or more and up to 20 years (Non Patent Literature 1). The high incidence of late recurrence, occurring even after 5 years after surgery, places a mental burden on patients.

In order to evaluate the risk of breast cancer recurrence, Oncotype DX (Exact Sciences Corp.) has been used for investigating 21 genes in surgery specimens. The risk evaluation by Oncotype DX is very useful for establishing a treatment plan, such as determining the effectiveness of postoperative anticancer drug treatment. However, Oncotype DX predicts the rate of recurrence within 10 years and cannot be said to be suitable for late recurrence cases. Despite the high number of late recurrence cases in the ER-positive type, there are no diagnostic markers that predict recurrent breast cancer 5 years after surgery. Prediction of a late recurrence risk leads to early detection of recurrence and early treatment. There is a need for proper diagnosis, prognosis prediction, and establishment of treatment for optimal medical treatment of breast cancers.

### Citation List

### Non Patent Literature

Non Patent Literature 1: Pan H., et al. N., Engl. J. Med. 2017, Vol.377, pp.1836-46.
Non Patent Literature 2: Tomita S., et al., Nat. Commun. 2015; 6: 6966.
Non Patent Literature 3: Yamamoto T., et al., Sci Rep 2018; 8: 15202.
Non Patent Literature 4: Abdalla M. O. A. et al., Nat. Commun. 2019; 10: 3778.
Non Patent Literature 5: Goss P.E., & Chambers A.F., Nat. Rev. Cancer 2010, Vol.10, pp.871-877.

### Summary of Invention

### Technical Problem

It is an object of the present invention to provide a method for selecting postoperative treatment by providing a test kit and test method for determining a late recurrence risk. In addition, since a marker correlating with late recurrence has been found, it is an object to provide a method for screening therapeutic drugs using the marker as a target.

### Solution to Problem

The present invention relates to the following test method, test kit, and method for screening therapeutic drugs:
(1) A method of assisting in testing for breast cancer late recurrence, the test-assisting method comprising detecting ELEANOR expression in a primary lesion, wherein when the ELEANOR expression is high, a risk of late recurrence is determined to be high.
   The present inventors have revealed that ELEANOR expression is an independent recurrence risk factor of breast cancer patients. When the ELEANOR expression is positive, the recurrence risk after 5 years after surgery is high.
(2) The test-assisting method according to (1), wherein estrogen receptor (ER) expression is detected.
   For ER-positive patients with a high risk of recurrence, it is recommended that postoperative endocrine therapy be extended from 5 years to 10 years after surgery. However, endocrine therapy causes adverse effects such as menopausal symptoms, joint pain, and thrombosis, and long-term treatment internally can also be stressful for patients. Accordingly, ELEANOR negativity can be used as a criterion for determining that extension of endocrine therapy is not necessary. This method can be a criterion for determining which patient needs extension and which does not.
(3) The test-assisting method according to (2), wherein progesterone receptor (PgR) and epidermal growth factor receptor type 2 (HER2) and Ki67 expressions are detected.
   In particular, since the late recurrence risk is high in a luminal B subtype, it can be more precisely determined whether endocrine therapy should be extended or not by examining the ELEANOR expression and factors that analyze these luminal subtypes.
(4) A test kit for testing a risk of breast cancer late recurrence, the test kit comprising a reagent for detecting ELEANOR expression.
(5) The test kit according to (4), wherein the reagent is a FISH probe and/or a PCR primer.
   As described above, since ELEANORS are risk factors of late recurrence of breast cancers, it is possible to detect a late recurrence risk of a breast cancer by examining ELEANORS by a FISH probe or a PCR primer.
(6) A method for screening breast cancer therapeutic drugs, the screening method comprising: culturing a cell expressing ELEANORS together with a candidate compound and using changes in ELEANOR expression and ER and/or CD44 expression as indicators to screen medicines.
(7) The screening method according to (6), wherein the cell expressing ELEANORS is an LTED cell.
   The present inventors revealed that the ELEANOR expression correlates with the late recurrence risk of a breast cancer and further correlates with ER expression and CD44 expression. Accordingly, screening compounds by using expressions of these molecules as indicators has a possibility of obtaining a compound that becomes a therapeutic drug for a late recurrent breast cancer. Furthermore, since the ELEANOR expression correlates also with the expression of a breast cancer stem cell marker CD44, a therapeutic drug that targets a breast cancer stem cell is likely to be obtained.
(8) A pharmaceutical composition comprising, as an active ingredient, a compound that is a therapeutic drug for a breast cancer and suppresses ELEANOR expression.
(9) The pharmaceutical composition according to (8), wherein the compound is a nucleic acid medicine.
   As shown below, the ELEANOR expression can be suppressed by a nucleic acid such as an siRNA and an LNA. Accordingly, these nucleic acids can become therapeutic drugs for late recurrence of breast cancers.
(10) A method for treating a late recurrence breast cancer, the method comprising: detecting ELEANOR expression in a primary lesion of a subject using the test-assisting method according to any one of (1) to (3) or the test kit according to (4) or (5), and performing treatment with the pharmaceutical composition according to (8) or (9) when a risk of late recurrence is determined to be high.

### Brief Description of Drawings

[Figure 1A] Figure 1A shows scoring of ELEANORS in 178 primary breast cancer cases. Score 1 and score 2 are defined as ELEANOR-positive.
[Figure 1B] Figure 1B shows a mosaic plot showing ratios of ELEANOR positive and negative patients in breast cancer subtypes. The figures in the plots show the real number of patients classified in each category (total n = 178) .
[Figure 1C] Figure 1C shows representative images of cases with ELEANOR scores of from 0 to 2 (from left to right): HE staining images (upper images), ER immunohistochemical staining images (middle images), and RNA FISH images (lower images) of corresponding samples.
[Figure 1D] Figure 1D shows mosaic plots of the relationship between the ELEANOR score and ER expression level in immunohistochemical staining analyzed by an Allred scoring system: correlations with the proportion score (left) and the intensity score (right). The Spearman's rank correlation coefficients are shown as rs.
[Figure 2A] Figure 2A shows Kaplan-Meier curves (n = 141, log-rank test) showing recurrence-free survival period (RFS, left) and overall survival period (OS, right) in ELEANOR positive and negative cases of patients with ER-positive primary lesions.
[Figure 2B] Figure 2B shows RFS of ELEANOR positive and negative patients with luminal A breast cancer (left) and luminal B breast cancer (right).
[Figure 2C] Figure 2C shows the Kaplan-Meier curves of Figure 2A by dividing into an early stage (left, from 0 to 5 years after surgery, or from 0 to 8 years after surgery) and a later stage (right, 5 or 8 years or more after surgery). ELEANOR-positive patients had poor prognosis in only the timeframe of the later stage (right) in both RFS (upper panel) and OS (lower panel), which are based on all data up to 5 or 8 years. The log-rank statistics after 5 years or 8 years after surgery are based on the data of all patients who were recurrence-free and alive at 5 years or 8 years later.
[Figure 3A] Figure 3A shows Kaplan-Meier curves (n = 139, Wilcoxon test) showing the presence or absence of ELEANORS in the primary lesions and the times until recurrence. "No. at Risk" shows the number of patients with a recurrence risk at each time.

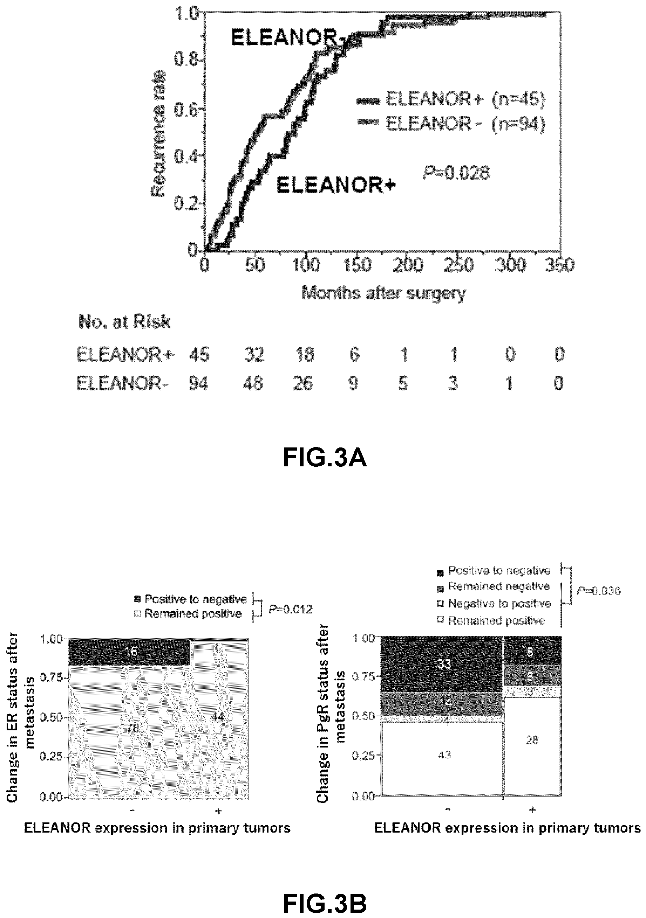

[Figure 3B] Figure 3B shows mosaic plots showing changes in the expressions of ER (left) and PgR (right) after metastasis. There are tendencies that in patients (-) who do not express ELEANORS in the primary lesions, the frequency of negative conversion of the expressions of ER and PgR is high and that in patients (+) who express ELEANORS in the primary lesions, the expressions of ER and PgR are maintained. The figures in the plots show the real number in each group (total n = 139). The P value is calculated from a Pearson's chi-square test and indicates the correlation between the ELEANOR expression in a primary lesion and the negative conversion of ER or PgR.
[Figure 4A] Figure 4A shows plots showing relationships between ELEANOR scores from 0 to 2 and expressions of ER by Allred scores: ER proportion scores (left) and intensity scores (right) in primary lesions (upper panel) and metastatic lesions (lower panel).
[Figure 4B] Figure 4B shows plots showing relationships between ELEANOR scores from 0 to 2 and expressions of PgR by Allred scores: PgR proportion scores (left) and intensity scores (right) in primary lesions (upper panel) and metastatic lesions (lower panel).
[Figure 5A] Figure 5A shows the results of sphere assay using HCC1428 and HCC1428-LTED cells: microscopic observation images (left) and their quantified graph (right).
[Figure 5B] Figure 5B shows FACS analysis of a stem cell marker CD44⁺/CD24^{-/low} in HCC1428-LTED cells in which ELEANOR2 has been knocked down (left), and its quantified graph (right).
[Figure 5C] Figure 5C shows a graph showing the results of RT-qPCR analysis of ESR1, ELEANOR2, pa-ELEANOR, and CD44 in HCC1428 and HCC1428-LTED cells.
[Figure 5D] Figure 5D is a graph showing the results of RT-qPCR analysis of expressions of ESR1 and CD44 when ELEANORS are knocked down by ELEANOR2 LNA and pa-ELEANOR LNA.
[Figure 5E] Figure 5E shows the results of immunoblotting analysis of CD44 expression caused by a change in ELEANOR expression (upper panel) and their quantified results (lower panel).
[Figure 5F] Figure 5F shows the results of FACS analysis of CD44⁺ cells of HCC1428-LTED cells after suppression of ELEANOR expression (left) and its quantified graph (right). The maximum value in the analysis is defined as 100.
[Figure 5G] Figure 5G shows a graph showing the results of RT-qPCR analysis of ELEANORS and CD44 mRNA after knock-down of ESR1.
[Figure 5H] Figure 5H shows the results of immunoblotting analysis of Erα expression in HCC1428-LTED cells after knock-down of ESR1, ELEANOR2, and pa-ELEANOR by siRNA or LNA.
[Figure 6A] Figure 6A shows the results of sphere assay of HCC1428-LTED cells and MCF7-LTED cells after knock-down of CD44: microscopic observation images (left) and their quantified graphs (right).
[Figure 6B] Figure 6B shows the results of analysis of propagation of HCC1428 cells or HCC1428-LTED cells transplanted into mammary fat pads of ovariectomized mice. Sizes of tumors (left, each group, n = 8) and tumors formed by transplantation of HCC1428-LTED cells (right, upper panel) are shown. HE staining, ER immunohistochemical staining, and ELEANOR RNA-FISH images are shown (right, lower panel). In mice transplanted with the parent strain HCC1428 cells, no tumors were formed.
[Figure 6C] Figure 6C shows the results of RT-qPCR analysis of ESR1 mRNA, ELEANORS, and CD44 mRNA in LTED cells before transplantation (in vitro) and after transplantation (in vivo).
[Figure 6D] Figure 6D shows RT-qPCR analysis of CD44 mRNA in various breast cancer cells.
[Figure 6E] Figure 6E shows the results of CD44 immunohistochemical staining after transplantation of each cell line into mice and shows representative images of respective cells. In Figures 6D and 6E, HCC1428, MDA-MB-231, and MDA-MB-468 cells were transplanted into mice without performing ovarian resection to form tumors in the cells.
[Figure 6F] Figure 6F shows RNA-FISH images of ELEANORS and centromere 6 (left) and ELEANORS and CD44 (middle). The CEN6 or CD44 signal and the ELEANOR signal overlap with each other were quantified (right). P values were calculated using a two-tailed Fisher's exact test.
[Figure 7A] Figure 7A shows HE staining images of tissues with ELEANOR scores of from 0 to 2 (upper panel), immunohistochemical staining images of CD44 (middle panel), and FISH analysis images of ELEANORS (lower panel).
[Figure 7B] Figure 7B shows mosaic plots showing relationships between ELEANOR scores and expression levels of CD44 in immunohistochemical staining. The proportion scores (left) and intensity scores (right) of CD44 were determined by an Allred scoring system. The figures in the plots show the real numbers. Spearman's rank correlation coefficients are shown as rs.

### Description of Embodiments

The group of the present inventors has studied a group of clusters of non-coding RNAs called ELEANORS (ESR1 locus enhancing and activating non-coding RNAs) for a long time. ELEANORS are a cluster of non-coding RNAs found in cells cultured under a long-term estrogen deprivation (LTED) condition, a model of an aromatase inhibitor therapy-resistant recurrent breast cancer. The LTED cells were established from ER-positive breast cancer cell lines such as MCF7 and HCC1428 (Non Patent Literature 2). ELEANORS are transcribed from a topological domain (topologically associating domain: TAD) of approximately 1 Mb (chr6: 151,750,000 to 152,750,000) including four genes such as an ESR1 gene encoding ER. In the nucleus, ELEANORS accumulate around the ESR1 gene to form an ELEANOR cloud and activate the entire ELEANOR TAD region. The present inventors have revealed that among the ELEANORS, u-ELEANOR, pa-ELEANOR, and ELEANOR2 are important for ELEANOR cloud formation and transcriptional activation of the genes in the ELEANOR TAD (Non Patent Literature 3).

The present inventors evaluated the clinicopathologic features of ELEANORS using primary and corresponding metastatic breast cancer tissues. As a result, it was revealed that expression of ELEANORS is limited to ER-positive cases and, in particular, well correlates with expression levels of ER and PgR in metastatic lesions. Furthermore, it was found that in patients who are ELEANOR-positive in primary lesions, the recurrence rate increases after 5 years, not within 5 years, unlike common cancers, and the present invention was accomplished.

The present invention will now be specifically described with reference to data, but is not limited thereto. The experimental procedures used below are based on those commonly used in the art unless otherwise stated.

### [Analysis object]

Analysis was performed using clinical specimens obtained from two independent cohorts. A cohort 1 is 185 primary breast cancer patients who underwent surgery for their tumors of 2 to 5 cm (pT2) in primary lesions in The Cancer Institute Hospital of JFCR during 2005 and 2006 and includes tumors of all pathological types. A cohort 2 is 167 cases of metastatic breast cancer patients who underwent surgery for ER-positive primary breast cancers during from 2001 to 2016 and then underwent biopsy or resection of the recurrent sites. In the cohort 2, analysis was performed using paired tissues of primary and metastatic sites.

### [ELEANOR cloud and ER expression in breast cancer tissue]

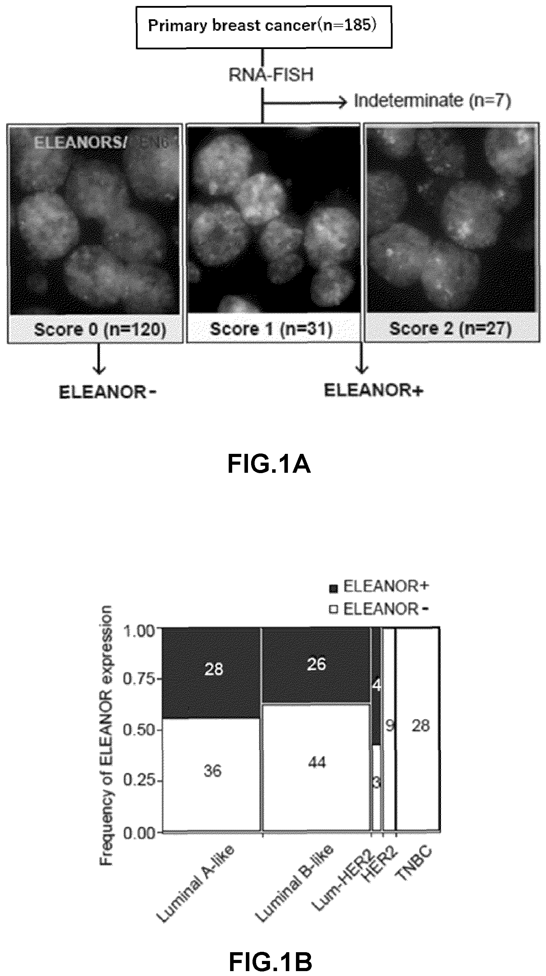

Although the chromatin control mechanism of ELEANORS in vitro has been studied, many aspects of the functions of ELEANORS in vivo are unknown. Accordingly, in order to investigate the clinical significance of ELEANORS, 185 primary breast cancer cases of the cohort 1 were analyzed by RNA-FISH to evaluate the ELEANOR expression (Figure 1A) .

The RNA-FISH was performed as follows. A 4-µm slice was produced from a resected or biopsy specimen, and ZytoLight FISH Tissue Implementation Kit (ZytoVision GmbH) was used. Specifically, a slide with the slice was heated at 70°C for 10 minutes, treated with xylene for 5 minutes three times, gradually rehydrated with an ethanol solution, washed with dH₂O, incubated with a citric acid solution of 98°C for 15 minutes, and washed with dH₂O. Subsequently, the slide was treated with a pepsin solution in a wet tray at 37°C for 30 minutes, then washed with 2 x SSC for 5 minutes, and subsequently washed with dH₂O for 1 minute. The slide was dehydrated and then air-dried. Subsequently, probes denatured by heating at 75°C for 10 minutes was added thereto. After hybridization at 37°C for 2 days, the slide was washed with a cleaning solution at 37°C for three times, dehydrated, and mounted with ProLong Diamond Antifade with DAPI (Thermo Fisher Scientific). As probes, A SPEC ESR1/CEN6 Dual Color Probe (ZytoVision GmbH) and BAC probes containing the ELEANOR locus (RP11-450E24) and the CD44 locus (RP11-790K21) were used.

For the detection of ELEANORS, the probes for detecting ELEANORS disclosed in Non Patent Literatures 2 to 4, specifically, probes detecting RP1-130E4, RP3-443C4, or a gene included in such regions, i.e., ESR1 or a gene present in the vicinity thereof, specifically, RP1-63I5, RP11-108N, or RP3-404G5, and although not described in these literatures, RP11-66L11 detecting ELEANOR2 can be used to detect ELEANORS. In addition to these probes, any probe can be used as long as it can specifically detect this region.

ELEANOR staining images by FISH were detected in the nuclei of cancer cells and classified into score 0, score 1, and score 2 from the smallest depending on the size of ELEANORS (Figure 1A). Furthermore, score 0 was defined as ELEANOR negative (-), and scores 1 and 2 were defined as ELEANOR positive (+). As a result of excluding 7 cases with specimens that were unsuitable for the FISH method, 58 cases (33%) were ELEANOR-positive tumors (Figure 1A).

ELEANOR expression was observed in ER-positive breast cancers of luminal A, luminal B, and luminal HER2 type (Figure 1B) and was not observed in ER-negative subtypes of HER2, and a triple negative (TNBC) type. Accordingly, focusing only on the ER-positive group (n = 141), it was demonstrated that the ELEANOR expression is significantly frequent in tumors from postmenopausal or over 50 years old patients (Table 1).

Herein, subtypes are defined as follows (Non Patent Literature 5): luminal A = ER and/or PgR positive, HER2 negative, Ki67 LI ≤ 14% or less, and grade < 3; luminal B = ER and/or PgR positive, HER2 negative, Ki67 LI ≥ 14%, grade 3; luminal HER2 = ER and/or PgR, HER2 positive; HER2 = ER and PgR negative, HER2 positive; and triple negative = ER, PgR, and HER2 negative.

**[Table 1]**

| Parameter | | ELEANOR expression, N (%) | | *P* value |
|---|---|---|---|---|
| | | Positive (n=58) | Negative (n=83) | |
| **Age** (years) | | | | <0.001** |
| | <50 | 11 (19%) | 40 (48%) | |
| | ≥50 | 47 (81%) | 43 (52%) | |
| **Menopausal status^{a}** | | | | 0.001** |
| | Premenopausal | 17 (29%) | 47 (57%) | |
| | Postmenopausal | 41 (71%) | 35 (43%) | |
| **Grade** | | | | 0.81 |
| | 1,2 | 43 (74%) | 60 (72%) | |
| | 3 | 15 (26%) | 23 (28%) | |
| **Ki67^{b}** | | | | 0.52 |
| | Low, Intermediate | 50 (88%) | 67 (84%) | |
| | High | 7 (12%) | 13 (16%) | |
| **PgR** | | | | 0.76 |
| | Positive | 46 (79%) | 64 (77%) | |
| | Negative | 12 (21%) | 19 (79%) | |
| **HER2** | | | | 0.21 |
| | Positive | 5 (9%) | 3 (4%) | |
| | Negative | 53 (91%) | 80 (96%) | |
| **ly** | | | | 0.53 |
| | Positive | 29 (50%) | 46 (55%) | |
| | Negative | 29 (50%) | 37 (45%) | |
| **Axillary lymph node metastasis** | | | | 0.90 |
| | Positive | 28 (48%) | 41 (49%) | |
| | Negative | 30 (52%) | 42 (51%) | |
| **No. of metastatic axillary lymph nodes** | | | | 0.44 |
| | 0-3 | 50 (86%) | 75 (90%) | |
| | 24 | 8 (14%) | 8 (10%) | |
| **Adjuvant therapy** | | | | |
| | Endocrine therapy^{c} | 53 / 57 (93%) | 69 / 81 (85%) | 0.16 |
| | Chemotherapy^{d} | 30 / 57 (53%) | 56183 (67%) | 0.08 |
| | Radiation therapy^{e} | 21 / 57 (37%) | 29183 (63%) | 0.82 |

| | | | | |
|---|---|---|---|---|
| One patient with unknown menopausal status^{a} and 4 patients with undetermined Ki67^{b} were excluded from the analysis. Three patients with unknown history of endocrine therapy^{c} and 1 patient with unknown history of chemotherapy^{d} and radiation therapy^{e} were excluded from the analysis. Statistically significant (***P* <0.01). | | | | |

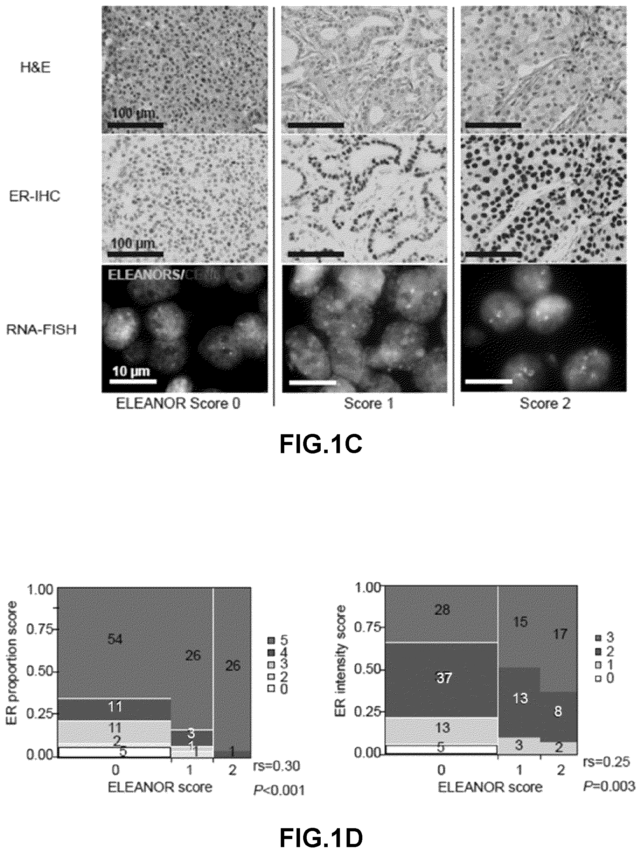

ER expression was detected by immunohistochemical staining, and a correlation with an ELEANOR cloud was analyzed (Figure 1C). In immunohistochemical staining (IHC), a formalin-fixed paraffin-embedded sample was sliced to 4 µm, and staining was performed with BenchMark ULTRA (Roche Diagnostics K.K.) or BOND-III (Leica Biosystems) automated slide staining system. When 1% or more of the cell nuclei are positive, it was defined as positive, and the expression level was evaluated using an Allred scoring system. As a result, it was revealed that ELEANOR clouds correlate with ER expression. A common Allred scoring system of ER using immunohistochemical staining demonstrated that the higher the ELEANOR score, the higher the proportion of ER-positive cells (ER proportion score) and the intensity of ER staining (ER intensity score) (respectively, rs = 0.30 and rs = 0.25, Spearman's rank correlation test, Figure 1D). These results are consistent with the observation results obtained in vitro previously that ELEANORS are present in LTED cells and ER is excessively expressed through a chromatin control function.

Immunohistochemical staining of PgR, CD44, HER2 were similarly performed, and the data thereof are shown below. The antibodies used are as follows: anti-ER antibody, clone SP1 (Roche Diagnostics K.K. or abcam. plc); anti-PgR antibody, clone 1E2 (Roche Diagnostics K.K.); anti-HER2 antibody, clone 4B5 (Roche Diagnostics K.K.); anti-Ki67 antibody, clone MIB-1 (DAKO); and anti-CD44 antibody, polyclonal (abcam. plc).

### [Correlation between ELEANOR expression and late recurrence of ER-positive breast cancer]

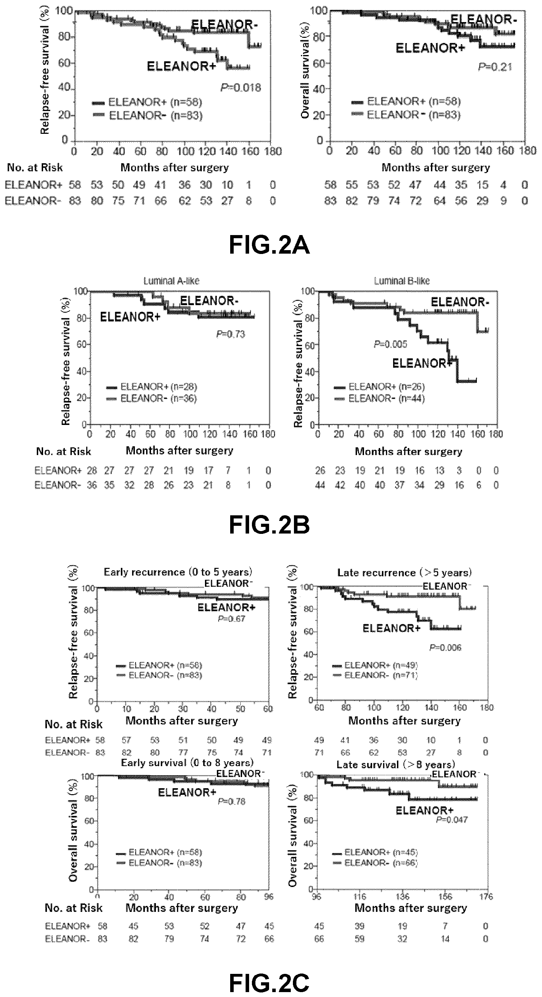

The ER-positive group (n = 141) of cohorts analyzed above was evaluated for recurrence-free survival period (RFS) and overall survival period (OS) (Figure 2A). It was demonstrated that in primary lesions, the RFS and the OS in the ELEANOR-positive patient group were shorter than those in the ELEANOR-negative patient group. In luminal A and luminal B breast cancers, the RFS of ELEANOR positive patients and the RFS of ELEANOR negative patients were analyzed. Among the ER-positive subtypes, the difference between the ELEANOR positive and negative was more significant in the luminal B type, but no difference was observed in the luminal A (Figure 2B).

The survival curve by a Kaplan-Meier method of Figure 2A was analyzed by dividing into timeframes of an early stage (RFS is from 0 to 5 years after surgery, and OS is from 0 to 8 years after surgery) and a later stage (RFS is 5 or more years after surgery, and OS is 8 years or more after surgery) (Figure 2C). It was demonstrated that the ELEANOR positive becomes a recurrence risk factor of the later stage (log-rank test, P = 0.006), but is not a recurrence risk of the early stage (log-rank test, P = 0.67) (Figure 2C, the upper panel). Similaraly, the results of the OS were that the later stage showed poor prognosis (Figure 2C, lower panel). Furthermore, though not shown here, worsening in the RFS after 5 years after surgery was observed in the patients with an ELEANOR score of 1 or 2, compared to the patients with an ELEANOR score of 0.

These results demonstrate that ELEANORS are involved in late recurrence of ER-positive breast cancers. Multivariable analysis with controlled disease stage and HER2 state was performed, and it was demonstrated that the ELEANOR expression is an independent recurrence risk factor of ER-positive breast cancer patients (Table 2, the hazard ratio (HR) = 2.40, P = 0.014). Furthermore, though not shown here, it was also demonstrated by analysis of a group of patients who underwent endocrine therapy that the ELEANOR-positive patient group has a high recurrence rate after 5 years after surgery. This result suggests that ELEANORS also play a significantly important role in acquiring resistance to postoperative endocrine therapy.

**[Table 2]**

| | | Multivariate analysis | |
|---|---|---|---|
| Variable | Univariate *P* value | Multivariate *P* value | Hazard ratio (95% CI) |
| ELEANOR status (negative/positive) | 0.017* | 0.014* | 2.40 (1.20-4.95) |
| Grade (1-2/3) | 0.034* | 0.11 | |
| HER2 status (negative/positive) | 0.021* | 0.42 | |
| No. of metastatic axillary lymph nodes (0-3/≥4) | 0.080 | | |
| Menopausal status (Pre-/Postmenopausal) | 0.43 | | |
| ly (negative/positive) | 0.66 | | |
| Ki67 (Low, Intermediate/High) | 0.72 | | |
| PgR status (negative/positive) | 0.93 | | |

| | | | |
|---|---|---|---|
| Cl: confidence interval. Statistically significant (**P* <0.05). | | | |

### [Analysis of metastatic lesion]

Involvement of ELEANORS in recurrence was analyzed using 167 pairs of ER-positive primary tumor and its corresponding metastatic lesion samples. ELEANOR expression was analyzed by FISH. The results of the analysis of remaining 139 cases excluding 28 cases that were not suitable for the FISH method are shown. It was demonstrated that ELEANORS were expressed in 45 cases (32%) of the primary lesions and 40 cases (29%) of the metastatic lesions. As in the results shown in Figure 2A, it was demonstrated that in these clinical sample sets, the period of time until the recurrence in the patients whose primary lesions were ELEANOR-positive was also longer than that in the group of ELEANOR-negative patients (Figure 3A). This demonstrates that ELEANORS are involved in late recurrence. In addition, the analysis of this data set also revealed that ELEANORS are excessively expressed by lung metastasis.

Changes in the ELEANOR expression in the primary lesion and recurrence lesion were analyzed by FISH for all patients (Table 3). The same state of ELEANORS as that in the primary lesion was maintained in the metastasis lesions in 116 cases (83%) out of 139 cases, and 31 cases (69%) out of 45 cases were ELEANOR positive even after metastasis. In patients after adjuvant therapy including aromatase inhibitor treatment, ELEANOR expression was slightly enhanced (Pearson's chi-square test, P = 0.1), although there is no significant statistical difference because of a small number of samples.

**[Table 3]**

| ELEANOR expression in primary tumors | ELEANOR expression in metastatic sites, N (%) | |
|---|---|---|
| | Negative (n=99) | Positive (n=40) |
| Negative (n=94) | 85 (90%) | 9 (10%) |
| Positive (n=45) | 14 (31%) | 31 (69%) |

It has been reported that the change in the state of hormone receptors (ER and PgR) after metastasis affects the prognosis and that the patients whose ER state became negative tend to show poor prognosis than the patients maintaining receptor positive. Accordingly, the relationship between changes in the expression of a hormone receptor and ELEANOR expression in primary lesions was analyzed. As a result, it was demonstrated that in a group of patients whose primary lesions are ELEANOR-positive, the frequency of conversion of the ER and PgR expression states from positive to negative after metastasis is low (Figure 3B, Pearson's chi-square test, P = 0.012, P = 0.036).

Subsequently, the expression states of ELEANORS and biomarkers of breast cancers in primary and metastatic lesions were analyzed (Table 4). The ELEANOR expression in the metastatic lesions significantly correlated with PgR positivity and HER2 negativity, but no correlation was observed in the primary lesions.

**[Table 4]**

| | | ELEANOR expression in primary tumors, N (%) | | | ELEANOR expression in metastatic sites, N (%) | | |
|---|---|---|---|---|---|---|---|
| Parameter | | Positive (n=45) | Negative (n=94) | *P* value | Positive (n=40) | Negative (n=99) | *P* value |
| **ER** | | | | - | | | <0.001** |
| | Positive | 45 (100%) | 94 (100%) | | 40 (100%) | 82 (83%) | |
| | Negative | 0 (0%) | 0 (100%) | | 0 (0%) | 17 (17%) | |
| **PgR** | | | | 0.91 | | | <0.001** |
| | Positive | 36 (80%) | 76 (81%) | | 31 (78%) | 47 (48%) | |
| | Negative | 9 (20%) | 18 (19%) | | 9 (23%) | 52 (53%) | |
| **HER2** | | | | 0.45 | | | 0.004** |
| | Positive | 5 (11%) | 15 (16%) | | 1 (3%) | 19 (19%) | |
| | Negative | 40 (89%) | 79 (84%) | | 39 (98%) | 80 (81%) | |
| **Grade** | | | | 0.90 | | | 0.55 |
| | 1, 2 | 33 (73%) | 68 (72%) | | 34 (85%) | 80 (81%) | |
| | 3 | 12 (27%) | 26 (28%) | | 6 (15%) | 19 (19%) | |
| **Ki67^{a}** | | | | 0.57 | | | 0.92 |
| | Low, Intermediate | 31 (70%) | 59 (66%) | | 30 (75%) | 72 (74%) | |
| | High | 13 (30%) | 31 (34%) | | 10 (25%) | 25 (26%) | |

| | | | | | | | |
|---|---|---|---|---|---|---|---|
| ^{a} Five primary cases and 2 metastatic cases with indeterminate Ki67 were excluded from this analysis. Statistically significant (***P* <0.01). | | | | | | | |

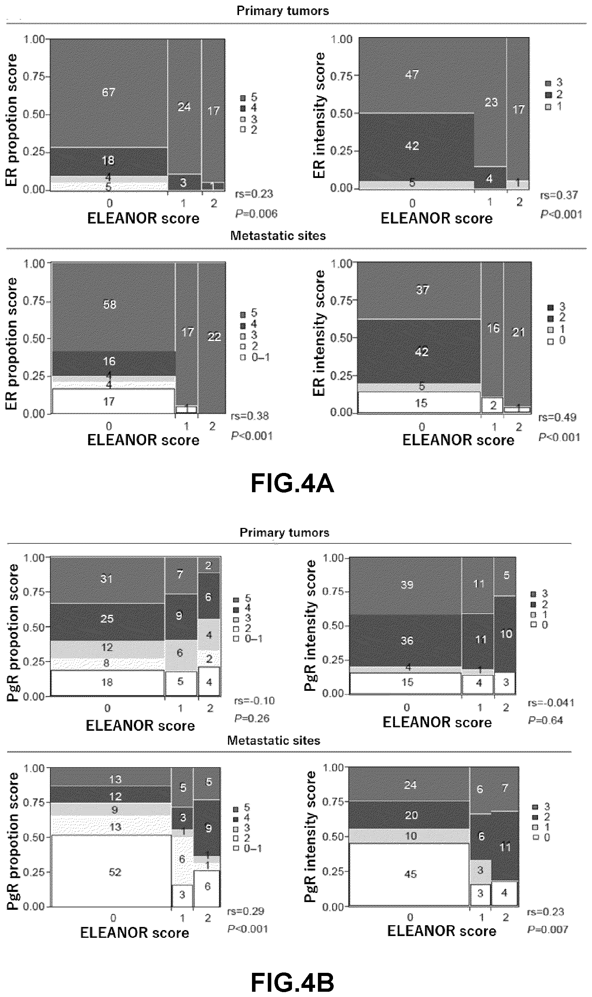

When detail immunostaining analysis was performed using an Allred scoring system, ELEANOR scores more strongly correlated with the ER proportion scores in the metastatic lesions (Figure 4A, left in the lower panel, rs = 0.38, P < 0.001) compared to those in the primary lesions (Figure 4A, left in the upper panel, rs = 0.23, P = 0.006). When the ER signal intensity was measured and analyzed (Figure 4A, right), it was demonstrated that the correlation between ELEANORS and ER was stronger in the metastasis lesions than in the primary lesions (metastatic lesion (Figure 4A, right in the lower panel), rs = 0.49; primary lesion (Figure 4A, right in the upper panel), rs = 0.37). Furthermore, in metastasis sites, a positive correlation was detected between ELEANORS and PgR (proportion score, rs = 0.29; intensity score, rs = 0.23) (Figure 4B, lower panel), but no correlation was observed in the primary lesion (Figure 4B, upper panel). These results suggest that ELEANORS activate the ESR1 gene as a transcription factor after metastasis and further activate target genes including the PgR gene. Accordingly, there is a possibility that ELEANORS are involved in metastasis of breast cancers through ER signaling pathway activation. These results suggest that a compound that suppresses ELEANOR expression can suppress metastasis and worsening.

### [Correlation between ELEANORS and CD44 expression and breast cancer stem cell]

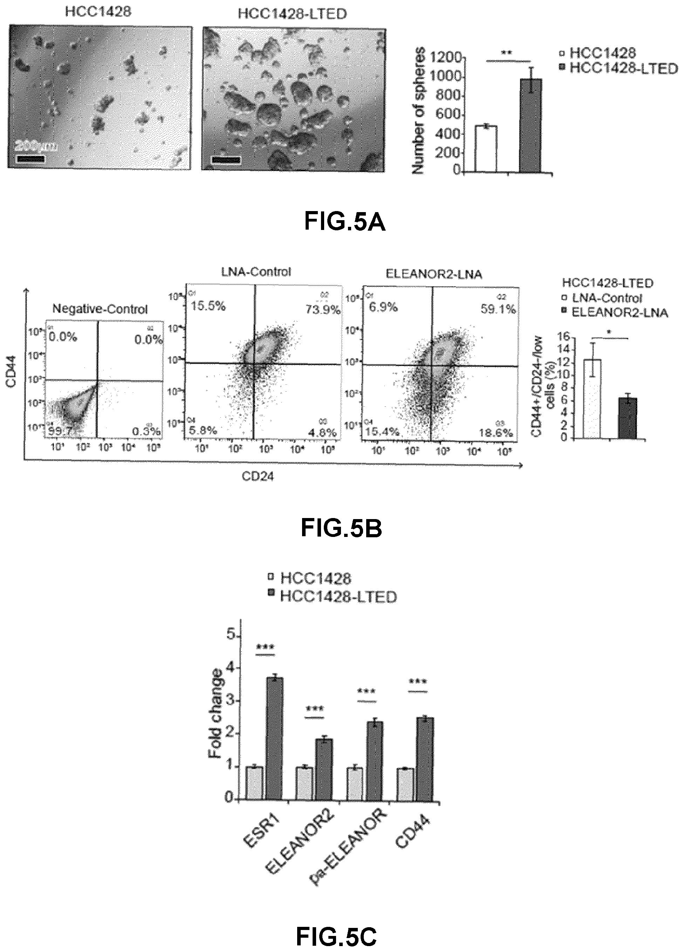

It is believed that the late recurrence of a breast cancer is caused by several factors such as tumor dormancy, cancer stemness, and endocrine therapy resistance. If ELEANORS are related in stemness, there is a possibility that suppression of ELEANOR expression leads to removal of breast cancer stem cells. Accordingly, the correlation between cancer stem cells and ELEANORS was analyzed. CD44⁺/CD24^{-/low} is known as a biomarker of a breast cancer stem cell. Breast cancer stem cells were analyzed using HCC1428 and MCF7 cell lines as ER-positive breast cancer models and LTED cells of these cell lines as endocrine therapy resistant ELEANOR high-expression in vitro models. Sphere assay was performed using the HCC1428 and HCC1428-LTED cells. Since the HCC1428-LTED cells showed higher sphere-forming ability compared to the HCC1428 cells, it was suggested that breast cancer stem cells are present in the HCC1428-LTED cells (Figure 5A).

ELEANOR2 RNA which is most abundant in ELEANORS was knocked down by LNA, and CD44/CD24 expression was analyzed by FACS (Figure 5B). The LNA below was transfected using RNAiMAX (Invitrogen), and analysis was performed after 48 hours. The number of breast cancer stem cells (CD44⁺/CD24^{-/low}) was significantly decreased by knocking down ELEANOR2.

ELEANOR2-LNA: TTTGGCCACATTGGAA (SEQ ID NO: 1)
LNA-Control: AACACGTCTATACGC (SEQ ID NO: 2)

Expressions of ESR1, ELEANORS, and CD44 in the HC1428 and HC1428-LTED cells were analyzed by RT-qPCR using the primers below. In the RT-qPCR, total RNA was purified from cultured cells using NucleoSpin RNA/protein kit (MACHEREY-NAGEL) and was reverse-transcribed by ReverTra Ace qPCR RT Master Mix (TOYOBO Co., Ltd.). The RT-qPCR was performed by ABI Prism 7500 (ABI inc.) using SYBR Green.

ESR1-S: CTCCTCCTCATCCTCTCCCA (SEQ ID NO: 3)
ESR1-AS: CTTTGGTCCGTCTCCTCCAC (SEQ ID NO: 4)
pa-ELEANOR-S: TAAGCCAATGTCAGGGCAAG (SEQ ID NO: 5)
pa-ELEANOR-AS: TCCCGAGCTCATATGCATTAC (SEQ ID NO: 6)
ELEANOR2-S: CGCCTCTGAAGTTTGCATC (SEQ ID NO: 7)
ELEANOR2-AS: GTCTGGGTGCCACTGTTTG (SEQ ID NO: 8)
CD44-S: CCCAGATGGAGAAAGCTCTG (SEQ ID NO: 9)
CD44-AS: GTTGTTTGCTGCACAGATGG (SEQ ID NO: 10)

For quantification of ELEANORS, probes of SEQ ID NOs: 11 to 40 disclosed in Non Patent Literatures 2 and 4 to detect ESR1, RMND1, C6orf211, or CCDC170 included in this region, can also be used. In addition, other probes can be similarly used, as long as they detect a gene or non-coding RNA included in this region.

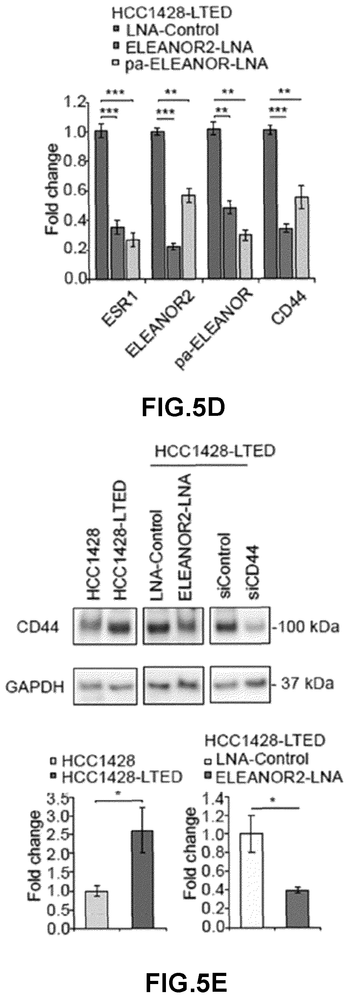

The results of the RT-qPCR analysis revealed that HCC1428-LTED cells excessively express CD44 mRNA and ELEANOR-specific sequences ELEANOR2 and pa-ELEANOR, compared to HCC1428 cells (Figure 5C). Subsequently, the ELEANOR expression was suppressed by LNAs of SEQ ID NO: 1 and SEQ ID NO: 41 specific to ELEANORS, and whether the CD44 expression is changed or not was investigated. The expression of CD44 mRNA was decreased by knock-down of these ELEANORS (Figure 5D).
pa-ELEANOR-LNA: GTGCCAGACTCCGATA (SEQ ID NO: 41)

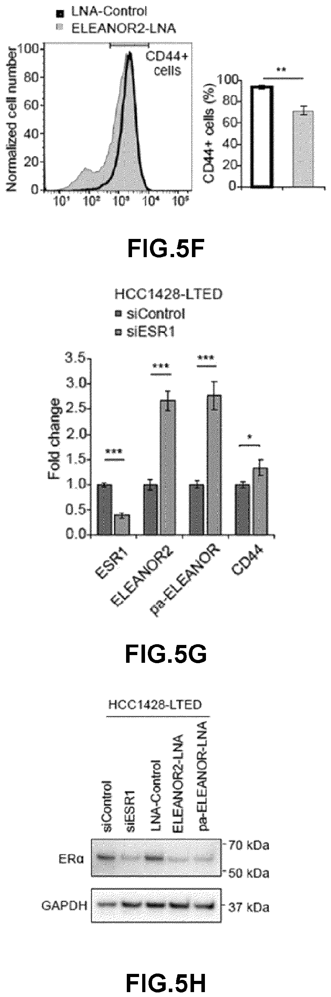

Whether the CD44 expression is changed also at the protein level was investigated. Comparative analysis of CD44 expression between HCC1428 and HCC1428-LTED cells was performed. The LNA of SEQ ID NO: 1 that suppresses ELEANOR expression and siRNA (Thermo Fisher Scientific) that suppresses CD44 expression were introduced into HCC1428-LTED cells, and the CD44 expression was analyzed by Western blotting (Figure 5E). The results of Western blotting analysis revealed that the expression of CD44 protein also correlates with ELEANOR expression. Furthermore, an ELEANOR was knocked down by introducing the LNA of SEQ ID NO: 1 into HCC1428-LTED cells, and whether the CD44 expression on the cell surface is also changed or not was analyzed by FACS. As a result, it was demonstrated that the number of CD44⁺ cells is decreased by knocking down the ELEANOR (Figure 5F).

Although data are not shown here, also in MCF7-LTED, the same results as above, i.e., high sphere-forming ability and high expressions of CD44 mRNA and protein were obtained in the LTED cells. However, although the effect of ELEANOR knock-down on CD44 expression in MCF7-LTED cells was significant at the mRNA level, the effect was almost not observed at the protein level. It is believed that this may be caused by the low CD44 protein level in MCF7-LTED cells. However, FACS analysis of MCF7-LTED cells demonstrated that the number of CD44⁺ cells was slightly but significantly decreased by ELEANOR2 knock-down. These data demonstrate that the functions of ELEANORS were preserved also in different cell lines and that ELEANORS are involved in the increase in the expression of the CD44 gene.

Subsequently, when the ESR1 expression was suppressed by siRNA (Sigma-Aldrich) of ESR1 to analyze the expressions of ELEANORS and CD44, no decrease in expression of any RNA was observed (Figure 5G). When the expressions of ESR1 and ELEANORS were knocked down to analyze the expression of ER, the ER expression was suppressed by knocking down any expression (Figure 5H). Despite suppressing the expression of ER protein by knock-down of ESR1 mRNA and knock-down of ELEANORS, the expression level of CD44 mRNA was not affected by mRNA knock-down of ESR1. These results eliminate the possibility of involvement of ER protein in activation of the CD44 expression in LTED cells.

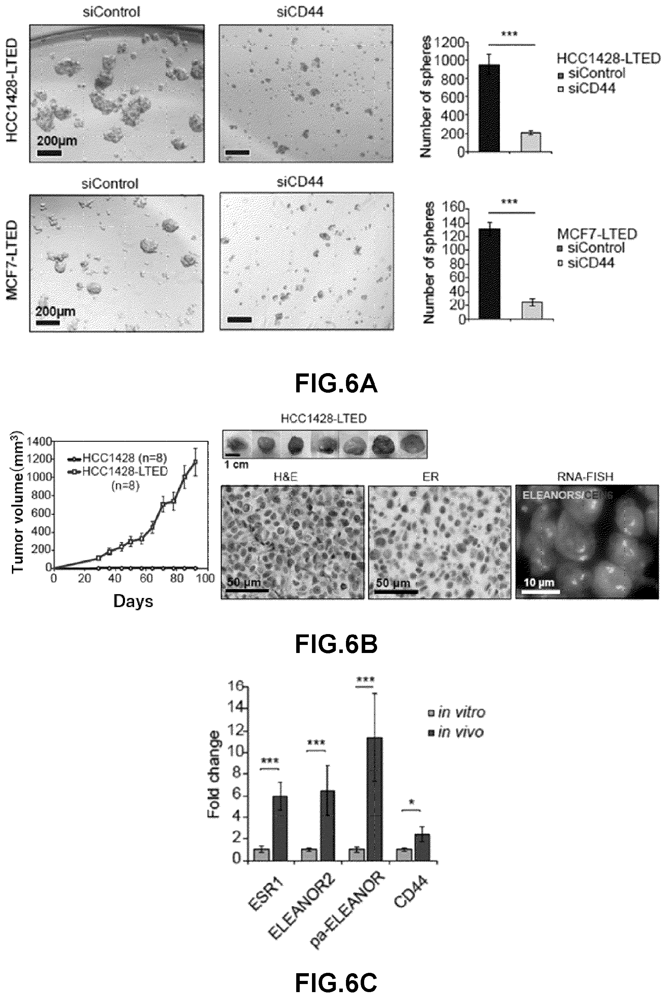

Furthermore, whether the up-regulation of CD44 by ELEANORS directly contributes to sphere-forming ability or not was investigated. When CD44 was knocked down by siRNA (Thermo Fisher Scientific) in LTED cells, it was found that the sphere-forming abilities of HCC1428-LTED and MCF7-LTED cells were significantly suppressed by CD44 knock-down (Figure 6A). These findings suggest that ELEANORS are involved in stem cell transformation through increasing the expression of CD44.

### [Analysis by xenograft]

In order to examine the functions of ELEANORS in vivo, HCC1428 cells and HCC1428-LTED cells were xenografted into mammary fat pads of ovariectomized NOD/SCID mice to evaluate tumorigenicity (each group n = 8). In every mouse transplanted with the parent strain HCC1428 cells, no palpable tumors were formed. In contrast, in mice transplanted with HCC1428-LTED cells, tumorigenesis was observed (Figure 6B, left, and right upper panel). It was confirmed by FISH that ELEANORS were expressed in the HCC1428-LTED cells transplanted into mice (Figure 6B, right lower panel). Furthermore, it was demonstrated by RT-qPCR that the CD44 mRNA level in HCC1428-LTED cells was increased in the cells after transplantation into mice, along with ESR1 and ELEANOR expressions, compared to the level in the cells cultured in vitro before transplantation (Figure 6C).

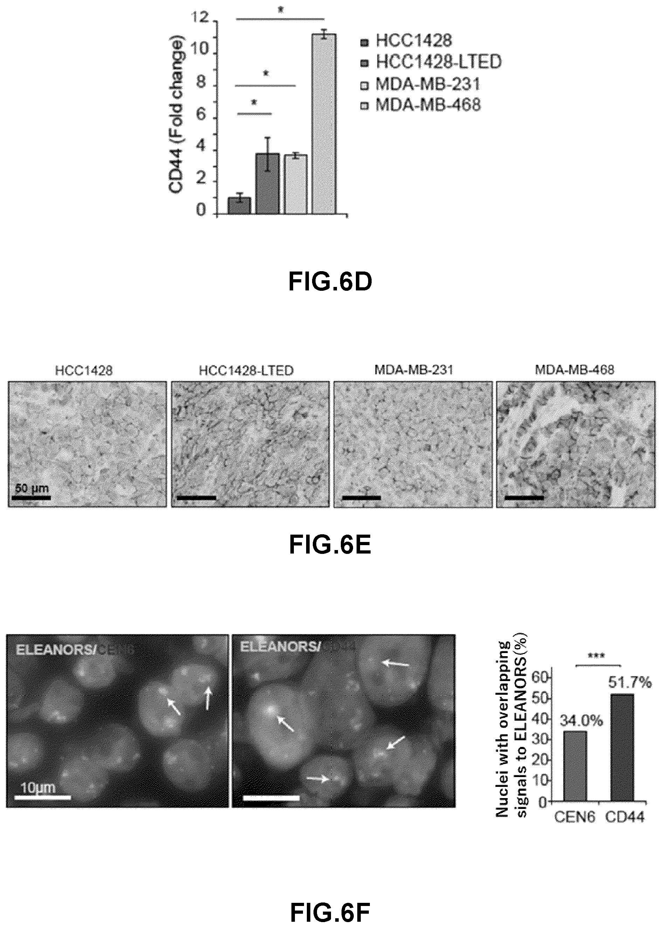

In addition to HCC1428 cells, MDA-MB-231 and MDA-MB-468 cells established from triple negative breast cancer patients were transplanted into mice without being ovariectomized, and HCC1428-LTED cells were transplanted into ovariectomized mice, and the CD44 expressions in tumors were analyzed (Figures 6D and 6E). In the tumors derived from HCC1428-LTED cells transplanted into ovariectomized mice, the expression levels of CD44 mRNA (Figure 6D) and protein (Figure 6E) were high compared to that in the tumors derived from HCC1428 cells transplanted into mice without being ovariectomized. In addition, the expression level of CD44 mRNA of the transplanted HCC1428-LTED was comparable to the expression of triple negative cell line MDA-MB-231 (Figure 6D), but at the protein level, the expression level was higher than that in the MDA-MB-231 cells and was the same level as those in other triple negative MDA-MB-468 cells (Figure 6E).

In order to investigate whether an increase in the expression of CD44 occurs near the ELEANOR cloud or not, ELEANORS and CD44 transcription sites were simultaneously visualized by RNA-FISH, using tumors derived from HCC1428-LTED cells that were xenografted into ovariectomized mice. As a result, the proportion of CD44 overlapping with ELEANORS and stained was high compared to the control centromere 6 (Figure 6F). It is believed from this that ELEANORS upregulate CD44 gene expression by providing a transcriptionally active nuclear environment.

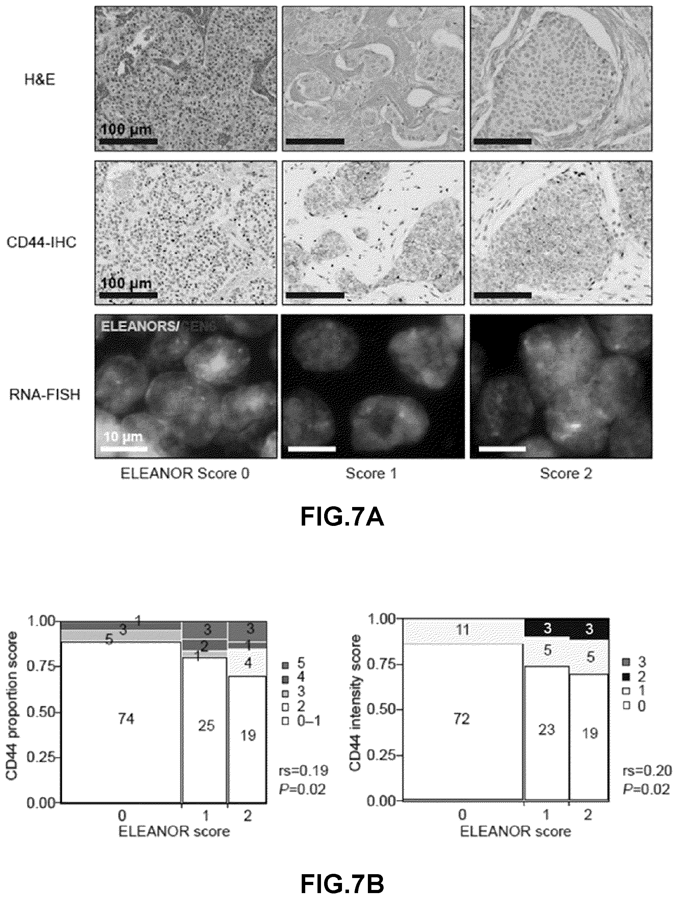

Subsequently, in order to investigate whether the CD44 expression state correlates with the ELEANOR expression in clinical specimens, CD44 expression in pT2 (2.0 cm < maximum diameter ≤ 5.0 cm) primary ER-positive breast cancer tissues (n = 141) in staging was analyzed. In a representative immunohistochemical staining analysis of CD44, more CD44-positive cells were observed in the ELEANOR-positive group (scores 1 and 2) (Figure 7A).

The Allred scoring system showed very weak correlations between the ELEANOR scores and the proportion and intensity scores of the CD44 expression (respectively, rs = 0.19, P = 0.02 and rs = 0.20, P = 0.02, Figure 7B). In addition to the analyses of cultured cell lines (Figure 5), the results obtained from the patients' tissues suggest the involvement of ELEANORS in late recurrence by maintaining the stemness of breast cancers through CD44 gene activation, in addition to ER signaling activation.

Based on the above results, a method of testing the risk of late recurrence, a test kit, and a method for screening medicines are provided. A specific test method is a method of detecting ELEANORS in a breast cancer primary lesion and determining the late recurrence risk to be high when an ELEANOR is positive. Conventionally, follow-up diagnosis for 5 to 10 years after surgery is performed, and treatment is completed in 5 years. However, when the late recurrence risk is high, measures for reducing the recurrence risk can be taken to prepare for recurrence, for example, follow-up observation after surgery is performed for a longer period of time such that recurrence can be detected and treated early, or treatment is continued beyond 5 years. In addition, for patients with a low risk of late recurrence, it is enough to perform follow-up diagnosis for 5 to 10 years after surgery as usual. Although tests for determining post-surgery treatment plans have traditionally existed, the present test method can identify patients with a high risk of late recurrence and is useful as a method of testing a risk of intractable recurrent breast cancers.

The test of the present invention may be any method as long as it can detect an ELEANOR. The method for detecting an ELEANOR may be detection by RNA-FISH, detection by RT-PCR of RNA specific to an ELEANOR such as ELEANOR2 and pa-ELEANOR, quantification by RT-qPCR, or detection of expression of a gene included in a topological domain of ELEANORS, such as ESR1, by RT-PCR or RT-qPCR at the RNA level or by ELISA, Western blotting, or the like at the protein level. A set of the reagents to be used in these tests, for example, FISH probes in RNA-FISH or primers in PCR, can be used to prepare a kit comprising reagents necessary for detection.

In addition, based on the above findings, it is possible to screen pharmaceutical compositions that treat late recurrent breast cancers or reduce the recurrence risk by using suppressed expression of ELEANORS as an indicator. Specifically, candidate compounds are added to culture solutions of cells, such as LTED cells, in which ELEANORS are highly expressed, and a compound that suppresses ELEANOR expression may be selected. In addition, siRNA, LNA, and so on that suppress ELEANOR expression can function as nucleic acid medicines.

## Claims

1. A method of assisting in testing for breast cancer late recurrence, the test-assisting method comprising detecting ELEANOR expression in a primary lesion, wherein when the ELEANOR expression is high, a risk of late recurrence is determined to be high.

2. The test-assisting method according to claim 1, wherein estrogen receptor (ER) expression is detected.

3. The test-assisting method according to claim 2, wherein progesterone receptor (PgR) and epidermal growth factor receptor type 2 (HER2) and Ki67 expressions are detected.

4. A test kit for testing a risk of breast cancer late recurrence, the test kit comprising a reagent for detecting ELEANOR expression.

5. The test kit according to claim 4, wherein the reagent is a FISH probe and/or a PCR primer.

6. A method for screening breast cancer therapeutic drugs, the screening method comprising: culturing a cell expressing ELEANORS together with a candidate compound and using changes in ELEANOR expression and ER and/or CD44 expression as indicators to screen medicines.

7. The screening method according to claim 6, wherein the cell expressing ELEANORS is an LTED cell.

8. A pharmaceutical composition comprising, as an active ingredient, a compound that is a therapeutic drug for a breast cancer and suppresses ELEANOR expression.

9. The pharmaceutical composition according to claim 8, wherein the compound is a nucleic acid medicine.
